# EUROPEAN PATENT APPLICATION

(11) **EP 2 584 340 A1**
(43) Date of publication of application: **24.04.2013**
(21) Application number: 11306358.0
(22) Date of filing: 20.10.2011
(51) Int. Cl.: G01N 21/35, G01N 21/77

(54) **Hydrogen sensing fiber and hydrogen sensor**

(71) Applicant: Draka Comteq BV, 1083 HJ Amsterdam (NL)
(72) Inventor: Burov, Ekaterina, 92100 Boulogne-Billancourt (FR); Pastouret, Alain, 91300 Massy (FR); Melin, Gilles, 91400 Orsay (FR)
(74) Representative: Hirsch & Associés

(57) **Abstract**

The invention concerns a hydrogen sensing fiber, pretreated so as to ease future diffusion to fiber core (11) of hydrogen (10) coming from outside the fiber (1). The invention also concerns a hydrogen sensor comprising a hydrogen sensing fiber (1) which is at least partly in contact with atmosphere coming from outside the hydrogen sensor (9).

## Description

### FIELD OF THE INVENTION

The invention relates to hydrogen sensing fibers and to hydrogen sensors including one or more hydrogen sensing fibers. Hydrogen detection, which is indeed molecular hydrogen detection, is particularly required because the leak and/or formation of gas in general, and the leak of hydrogen in particular, often is an indicator of chemical activity such as water radiolysis or metallic structure corrosion.

### BACKGROUND OF THE INVENTION

Some prior art hydrogen sensors do not include hydrogen sensing fibers because they rely on other technologies. Such prior art hydrogen sensors often do not present the same flexibility than hydrogen sensors based on hydrogen sensing fibers.

Some prior art hydrogen sensors include hydrogen sensing fibers. Such prior art hydrogen sensors already present some advantages, notably their ability to be distributed and deported sensing systems. However, such prior art hydrogen sensors, as will be discussed below, are not as reliable and/or flexible as possible, because they often present one or more of the following drawbacks: hydrogen presence detection which is not precise enough, hydrogen presence detection which does not stay reliable over extended periods of time, hydrogen presence detection which presents too long response time, too much complexity.

According to a first prior art, for example described in international application WO2009154216 (A1), it is disclosed a hydrogen fiber sensor system in which hydrogen detection is realized thanks to a detection layer at the periphery of the fiber containing a platinum catalyst whose refractive index is modified by the presence of hydrogen. This first prior art discloses a very complicated and costly fiber structure based on coating the fiber with Platinum and tungsten oxide layers.

According to a second prior art, for example described in international application WO2009067671 (A1), it is disclosed a hydrogen fiber sensor system in which hydrogen detection is realized thanks to modification of fiber characteristics at one or more wavelengths in the presence of hydrogen. This second prior art discloses a non reliable over time and with a long response time, especially in environments in which the temperature can vary in a large scale and in a not predictable way, notably because the phenomenon used is the irreversible increase of attenuation due to irreversible increase of OH peak, for example at 1380nm.

According to a third prior art, for example described in international application WO 2003/056313 A1, it is disclosed an irreversible reaction with a metallic catalyst on fiber cladding, which increases fiber attenuation. This third prior art discloses a complicated and costly fiber structure based on use of a metallic catalyst.

According to a fourth prior art, for example described in international application US 5,153,931, it is disclosed a hydrogen presence detection by hydrogen adsorption on hydrogen sensing fiber cladding. This fourth prior art discloses a hydrogen sensing fiber structure based on hydrogen adsorption of fiber cladding which can only detect hydrogen presence without quantifying it in a reliable way.

### SUMMARY OF THE INVENTION

The object of the present invention is to alleviate at least partly the above mentioned drawbacks.

More particularly, the invention aims at providing a hydrogen sensor including hydrogen sensing fiber, which improves the global compromise between hydrogen presence detection precision, hydrogen presence detection reliability over time, hydrogen presence detection shortened response time, while keeping a relative simplicity in hydrogen sensor structure, and particularly in hydrogen sensing fiber structure.

To improve precision, some embodiments of the invention preferably aim at detecting hydrogen presence through a modification of a property of hydrogen sensing fiber which is the more possible representative of hydrogen concentration in the vicinity of hydrogen sensor, then allowing not only for a simple hydrogen presence detection but also for real quantification of hydrogen concentration. Advantageously, such a measured property is fiber attenuation, whose fiber attenuation is most directly impacted by hydrogen diffusion in fiber core.

To increase duration of reliability, some embodiments of the invention preferably aim at detecting hydrogen presence in a reversible way, so that theoretical life time of hydrogen sensor is more limited by life time of hydrogen sensing fiber than by quantity of hydrogen in hydrogen sensor vicinity which can, if too high over extended periods of time, for some prior art sensors, saturate prematurely the hydrogen sensing fiber. Advantageously, hydrogen interaction with hydrogen sensing fiber to modify sensing fiber property will be chosen as a reversible interaction; when hydrogen comes and then disappears, hydrogen sensing fiber property is modified and then comes back to original value.

To shorten response time, some embodiments of the invention preferably aim at favoring hydrogen effect on hydrogen sensing fiber, and even more preferably at accelerating hydrogen effect on hydrogen sensing fiber, so that hydrogen presence detection occurs more quickly. Advantageously, hydrogen effect on hydrogen sensing fiber is essentially due to hydrogen presence in hydrogen sensing fiber core after hydrogen diffusion into hydrogen sensing fiber core.

A first object of the invention is achieved with a hydrogen sensing fiber, comprising a core and a cladding, wherein the fiber is pretreated with a gas able to react with glass defects present in the untreated fiber so that the fiber does not exhibit irreversible hydrogen induced losses at any wavelength after exposure of at least a part of the fiber in an atmosphere comprising Hydrogen.

A second object of the invention is achieved with a Hydrogen sensor comprising a hydrogen sensing fiber according to some embodiments of the invention, said hydrogen sensing fiber being at least partly in contact with an atmosphere coming from outside the hydrogen sensor.

A third object of the invention is achieved with a Hydrogen detecting method, comprising a step of launching light in a hydrogen sensing fiber according to some embodiments of the invention included in a hydrogen sensor, at a first wavelength where said hydrogen sensing fiber can still undergo reversible hydrogen induced losses, a step of detecting light coming out from said hydrogen sensing fiber, at said first wavelength, a step of calculating fiber attenuation at said first wavelength by comparing detected light power to launched light power at said first wavelength, a step of quantifying, directly or indirectly, hydrogen presence in outside atmosphere surrounding the hydrogen sensor from calculated fiber attenuation.

A fourth object of the invention is achieved with a Hydrogen sensing fiber pretreatment method, successively comprising a step of exposing said hydrogen sensing fiber, to an atmosphere including a predetermined Hydrogen concentration, under a predetermined pressure, at a predetermined temperature, during a first predetermined time, a step of out gazing at ambient conditions during a second predetermined time.

Preferred embodiments comprise one or more of the following features:
- said gas is Hydrogen.
- fiber core is pure silica or silica doped with fluorine.
- fiber cladding thickness ranges from 5µm to 30µm.
- fiber cladding is made of polymer material.
- said hydrogen sensing fiber is a multimode fiber.
- Hydrogen sensor further comprising a light source adapted to launch light in said hydrogen sensing fiber, at a first wavelength within a spectral range of a reversible attenuation increase due to hydrogen presence in the fiber core, a light detector adapted to detect light coming out from said hydrogen sensing fiber, at said first wavelength.
- said light source and said light detector are encompassed in an optical time-domain reflectometer.
- said first wavelength is about 1242nm.
- Hydrogen sensor further comprising a heater adapted to heat said hydrogen sensing fiber.
- Hydrogen sensor further comprising a calculator adapted to calculate fiber attenuation by comparing detected light power to launched light power at said first wavelength and to quantify, directly or indirectly, hydrogen presence in an atmosphere surrounding the hydrogen sensor from the calculated fiber attenuation.
- said calculator is further adapted to calculate the difference between fiber attenuation at said first wavelength and fiber attenuation at a second wavelength, said hydrogen sensing fiber presenting at said second wavelength an attenuation independent of quantity of hydrogen present in the fiber core, and adapted to quantify hydrogen presence in an atmosphere surrounding the hydrogen sensor from said calculated difference.
- Hydrogen sensor further comprising a temperature sensor coupled to said hydrogen sensing fiber and providing said calculator with a signal representing the temperature of said hydrogen sensing fiber, and wherein said calculator is adapted to correct calculated fiber attenuation(s) according to hydrogen sensing fiber temperature variations.
- the length of the hydrogen sensing fiber is at least 100m, preferably at least 500m, more preferably at least 1000m.
- for at least one point of the hydrogen sensing fiber, the lowest from two distances between said point and respectively source and detector, is at least 5m.
- Hydrogen detecting method further comprising a step of heating the hydrogen sensing fiber so that hydrogen sensing fiber temperature exceeds temperature of outside atmosphere surrounding the hydrogen sensor by at least 10°C, preferably by at least 20°C.
- Hydrogen detecting method further comprising a step of heating the hydrogen sensing fiber so that a 1% hydrogen presence in the atmosphere surrounding the hydrogen sensor can be detected and quantified in less than 1 hour, preferably in less than 10 minutes.
- the atmosphere surrounding the hydrogen sensor is radioactive.
- the predetermined pressure ranges preferably from 1 to 10 atmospheres, more preferably from 2 to 10 atmospheres, the predetermined temperature ranges preferably from 20°C to 150°C, more preferably from 50°C to 90°C, the first predetermined time ranges preferably from 30 hours to 1000 hours, the second predetermined time is preferably at least 2 weeks.
- predetermined hydrogen concentration either ranges from 1% to 4% or ranges from 90% to 100%.

Further features and advantages of the invention will appear from the following description of embodiments of the invention, given as non-limiting examples, with reference to the accompanying drawings listed hereunder.

### BRIEF DESCRIPTION OF THE DRAWINGS

Fig. 1 shows an example of a hydrogen sensing fiber according to an embodiment of the invention.
Fig. 2 shows an example of a sensor including in its casing a hydrogen sensing fiber according to an embodiment of the invention.
Fig. 3 shows another example of a distributed sensor including a hydrogen sensing fiber according to an embodiment of the invention.
Fig. 4 shows an example of a hydrogen presence detecting method according to an embodiment of the invention.
Fig. 5 shows an example of a hydrogen sensing fiber pretreatment method according to an embodiment of the invention.
Fig. 6 shows an example of curves showing fiber attenuation increase as a function of time due to hydrogen diffusion in fiber core in different conditions.
Fig. 7 shows an example of curves showing hydrogen presence detection as a function of time due to hydrogen diffusion in fiber core for different temperatures of hydrogen sensing fiber.
Fig. 8 shows an example of curves showing hydrogen diffusion time in fiber core as a function of both hydrogen sensing fiber temperature and hydrogen sensing fiber cladding thickness.
Fig. 9 shows another example of a hydrogen sensing fiber according to an embodiment of the invention.
Fig. 10 shows still another example of a hydrogen sensing fiber according to an embodiment of the invention.

### DETAILED DESCRIPTION OF THE INVENTION

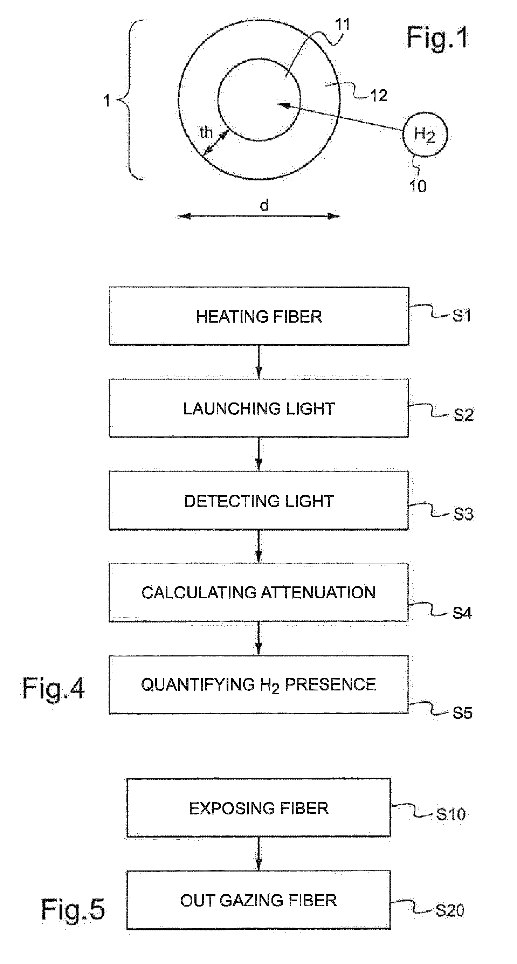

Fig. 1 shows an example of a hydrogen sensing fiber according to an embodiment of the invention. Hydrogen sensing fiber 1, more commonly called sensing fiber 1 or even fiber 1, presents a fiber core 11 and a fiber cladding 12. Sensing fiber 1 may also present one or more fiber coatings around fiber cladding 12 to be better protected. Sensing fiber 1 presents a diameter d and a fiber cladding thickness th. When hydrogen 10 is present in the vicinity of sensing fiber 1, for example when hydrogen 10 surrounds sensing fiber 1, hydrogen 10 will diffuse with time from outside the sensing fiber 1 through the sensing fiber 1, first through fiber coating(s) if any, then through fiber cladding 12, and finally in fiber core 11 toward fiber core center.

Sensing fiber 1 has been pretreated so as to ease future diffusion to fiber core 11 of hydrogen 10 coming from outside the sensing fiber 1. Indeed, sensing fiber 1 property measured is sensing fiber 1 attenuation, is mostly dependent on fiber core 11 state. So, the more quickly hydrogen 10 can diffuse until it is present in fiber core 11 and preferably in all fiber core 11, especially in fiber core 11 center, the shortest is the response time of hydrogen sensing fiber 1 and associated hydrogen sensor including this hydrogen sensing fiber 1.

Sensing fiber 1 has been pretreated until it cannot undergo further irreversible hydrogen induced losses at any wavelength, preferably during sensing fiber 1 whole lifetime. Indeed, if all possible irreversible interactions between sensing fiber 1 and hydrogen 10 have already occurred during a pretreatment phase, then, when sensing fiber 1 is in use, hydrogen 10 will diffuse more quickly in fiber core 11, and more quickly towards fiber core center, because it will no more be stopped by defects present in fiber cladding 12 and/or in fiber core 11, which would block hydrogen diffusion during a while by interacting with part of hydrogen molecules.

Sensing fiber 1 has been pretreated under a gas which is able to react with existing fiber glass defects. Preferably, all fiber defects will be saturated in advance. Then, later on, when sensing fiber 1 will be in use, those defects will no more curtail hydrogen diffusion, because those defects having already reacted with said gas, will no more be able to react with hydrogen coming from outside. Advantageously, to be sure that all defects which can react with hydrogen have already reacted in an irreversible way, the best is to make these defects react with hydrogen in advance, that is indeed during a pretreatment phase. Deuterium would not be a suitable gas for pretreatment until sensing fiber 1 cannot undergo further irreversible hydrogen induced losses, since hydrogen can replace deuterium at plots of fiber defects, and then deuterium diffusion, instead of hydrogen diffusion, towards fiber core center would not have the same effect on fiber attenuation increase that would have had hydrogen diffusion instead.

Fiber core 11 is preferably pure silica or silica doped with fluorine. Such a doped fiber core 11 is more robust in harsh environments like environments where outside atmosphere surrounding the hydrogen sensor is radioactive. For example, some storage places exist underground to store radioactive waste. When, in those storage places, there is a hydrogen leak, it means that there is a problem. Either there is a corrosion of a metallic container, or there is a reaction between radioactive waste and outside environment due to a leakage in container of radioactive waste. Besides, above a level of 4% hydrogen concentration in outside air, there is a non negligible risk of explosion. Another example of such a radioactive environment can be a nuclear power station. Therefore, in such radioactive environments, hydrogen sensing fiber 1 is already interesting because of shortened response time, response time being here a rather critical parameter, and particularly interesting when it is very robust. Doping fiber core 11 with fluorine or non doping fiber core 11 improves robustness with respect to doping fiber core 11 with germanium which is much more sensitive to radiations.

In order to ease more hydrogen diffusion in fiber core 11, the thickness th of fiber cladding 12 can be reduced. In this way, hydrogen diffuses though fiber cladding 12 more quickly. A compromise has to be found, because if there is no more fiber cladding 12 or too little, fiber core 11 is no more sufficiently protected. Therefore, fiber cladding 12 thickness th preferably ranges from 5µm to 30µm. For similar reason, fiber cladding 12 is preferably made of plastic material, rather than silica, and especially of polymer material. Hydrogen diffuses more easily and more quickly through polymer than through silica, because of the lower density of polymer compared to density of silica.

Sensing fiber 1 can be a multimode fiber with a large core diameter d, for example about 50µm or more, or a single mode fiber with a small core diameter, for example about 10µm or less. A multimode sensing fiber 1 is preferred, because it will need no coating or less coating than a single mode fiber. A single mode fiber will need coating and thicker coating due to intrinsic brittleness. Presence of coating will slow down hydrogen diffusion. A multimode sensing fiber 1 without coating (but with a fiber cladding 12) is preferred.

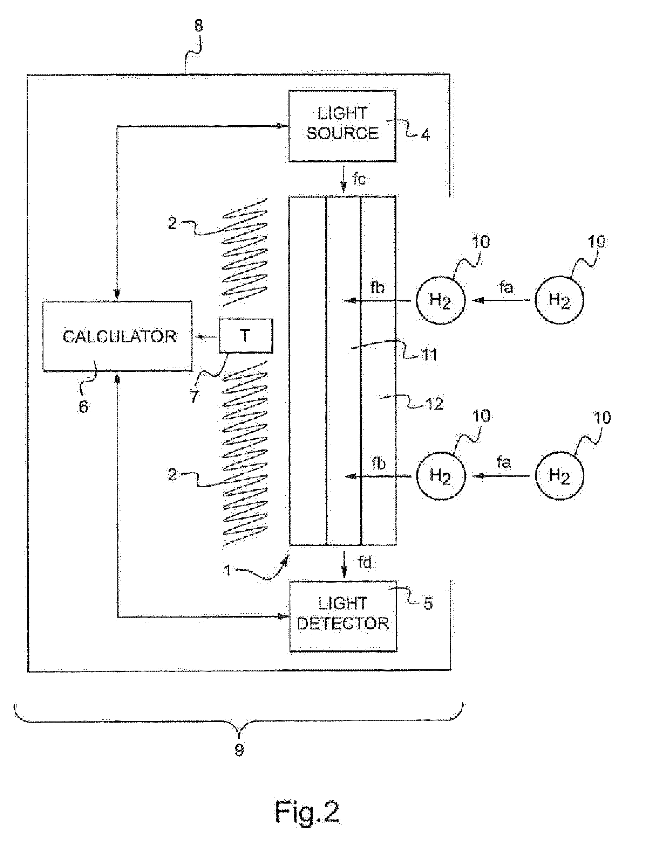

Fig. 2 shows an example of a sensor including in its casing a hydrogen sensing fiber according to an embodiment of the invention. In the casing 8 of a hydrogen sensor 9, there are hydrogen sensing fiber 1, heater 2, light source 4, light detector 5, calculator 6, temperature sensor 7.

The light source 4 is adapted to launch light in sensing fiber 1, at a first wavelength where sensing fiber 1 can still undergo reversible hydrogen induced losses. The light detector 5 is adapted to detect light coming out from sensing fiber 1, at the same first wavelength.

Outside the sensor 9, which is to say here outside its casing 8, there is the outside environment including hydrogen 10a. This hydrogen 10a moves from outside the casing 8 into the casing 8. This air circulation is shown by arrow fa. Then, in the casing 8, there is hydrogen 10b which will diffuse in sensing fiber 1, first through fiber cladding 12, then through fiber core 11. This diffusion of hydrogen 10b in sensing fiber 1 is shown by arrow fb. In that way, sensing fiber 1 is in contact with atmosphere coming from outside the hydrogen sensor 9.

Light source 4 launches light into the fiber core 11 at one end of sensing fiber 1. Launched light is shown by arrow fc. All along sensing fiber 1, this light propagates until coming out at the other end of sensing fiber 1. By detecting light coming from sensing fiber 1 at light detector 5 and analyzing it in calculator 6, fiber attenuation can be computed. Detected light is shown by arrow fd. Fiber attenuation increase will be the indicator of hydrogen presence in fiber core 11. The amount of fiber attenuation increase leads to hydrogen concentration value.

In order to make more precise the measurement of fiber attenuation increase due to hydrogen presence in fiber core 11, the first wavelength is chosen within spectral range of a reversible attenuation peak due to hydrogen presence in fiber core 11. Preferably the peak in wavelength range corresponding to first overtone of resonance frequency of hydrogen is chosen because this is the highest peak among all overtones, and by far, so the one leading to the more precise fiber attenuation increase computation.

Peak of first overtones is preferable to peak of fundamental because peak of fundamental is at a wavelength of more than 2µm, which is not in the "transmission window" of silica, that is to say peak of fundamental lies in a spectral range where attenuation being intrinsically very high, detecting a fiber attenuation increase would be more difficult and would lead to less precise fiber attenuation measurement, turning out into less precise hydrogen concentration quantification. Advantageously, first wavelength is about 1242nm, for example 1242nm more or less 2nm, first overtone peak ranging approximately from 1240nm to 1244nm.

Sensor 9 comprises a heater 2 which is adapted to heat sensing fiber 1. This heater 2 is for example disposed around sensing fiber 1. Heating sensing fiber 1 has a first positive effect on sensor response time shortening, because it accelerates hydrogen diffusion to fiber core center. Heating sensing fiber 1 has a second negative effect on hydrogen quantification precision, because it reduces the level of the reversible attenuation peak due to hydrogen presence in fiber core 11. The first positive effect on sensor response time outweighs the second negative effect on quantification precision, at least over a wide temperature range, and outweighs largely over a more limited temperature range therefore preferred.

Advantageously, heater 2 is adapted to heat permanently sensing fiber 1, when sensor 9 is in use. Heater 2 may also be adapted to heat intermittently sensing fiber 1, when sensor 9 is in use, if hydrogen concentration measures are only needed at long time intervals in considered application.

Sensor 9 comprises a calculator 6 which is adapted to calculate fiber attenuation by comparing detected light power to launched light power at first wavelength. Calculator 6 is also adapted to quantify, directly or indirectly, hydrogen presence in outside atmosphere surrounding the sensor 9 from calculated fiber attenuation.

To make measurements more precise, in an environment where outside conditions can vary quickly and deeply, calculator 6 is preferably adapted to calculate difference between fiber attenuation at first wavelength and fiber attenuation at a second wavelength, said hydrogen sensing fiber presenting, at said second wavelength, attenuation which is independent of quantity of hydrogen present in fiber core 11. So, at second wavelength, there is no reversible hydrogen induced losses, but there could be irreversible hydrogen induced losses. Calculator 6 is then also adapted to quantify hydrogen presence in outside atmosphere surrounding the sensor 9 from said calculated difference.

To make measurements even more precise, in an environment where outside temperature can vary quickly and deeply, hydrogen sensor 9 further comprises a temperature sensor 7 coupled to hydrogen sensing fiber 1 and to calculator 6 so as to provide calculator 6 with temperature of hydrogen sensing fiber 1. Calculator 6 is then adapted to correct calculated fiber attenuation(s) according to hydrogen sensing fiber 1 temperature variations.

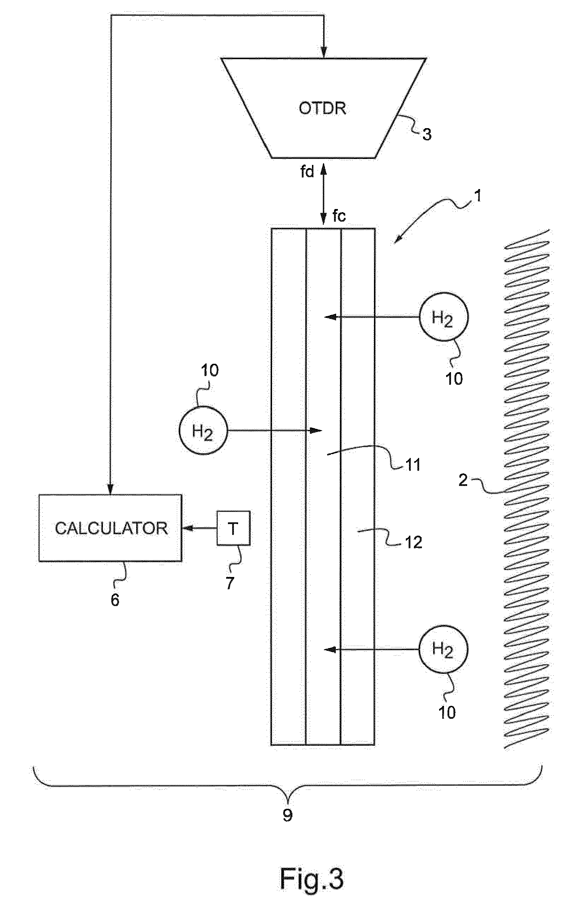

Fig. 3 shows another example of a distributed sensor including a hydrogen sensing fiber according to an embodiment of the invention. Hydrogen sensor 9 comprises hydrogen sensing fiber 1, heater 2, an optical time-domain reflectometer 3 more simply called OTDR 3, calculator 6, temperature sensor 7.

OTDR 3 encompasses a light source and a light detector. The light source is adapted to launch light in sensing fiber 1, at a first wavelength where sensing fiber 1 can still undergo reversible hydrogen induced losses. The light detector is adapted to detect light coming out from sensing fiber 1, at the same first wavelength. Hydrogen 10 diffuses into fiber core 11 the same way as in figure 2.

OTDR 3 launches laser pulses into the fiber core 11 at one end of sensing fiber 1. Launched light is shown by arrow fc. All along sensing fiber 1, those laser pulses are reflected by Rayleigh back scattering. By detecting back scattered laser pulses in OTDR 3 coming out of sensing fiber 1 at same end where it was launched, and by analyzing them in calculator 6, fiber attenuation can be computed. Detected light is shown by arrow fd. Fiber attenuation increase will be the indicator of hydrogen presence in fiber core 11. Optical time-domain reflectometry can give the position of fiber attenuation increase in sensing fiber 1 leading from there to the precise localization of hydrogen presence along the hydrogen sensing fiber. The amount of fiber attenuation increase leads to hydrogen concentration value.

In an example of hydrogen sensor 9, the length of hydrogen sensing fiber 1 is at least 100m, preferably at least 500m, more preferably at least 1000m. Then, wide areas can be monitored by hydrogen sensor 9. In that way, we can have a distributed sensor 9 over a more extended area. Preferably, for at least one point of hydrogen sensing fiber 1, the lowest from two distances between said point and respectively source 4 and detector 5, is at least 50m, preferably at least 200m, more preferably at least 500m. In case of an OTDR 3 encompassing source 4 and detector 5, for at least one point of hydrogen sensing fiber 1, the distance between said point and OTDR 3, is preferably at least 5m. Optical time-domain reflectometry usually needs a minimal length of sensing fiber 1 which is rather long to be very precise. If used in small hydrogen detection area, one can wound optical fiber in order to use optical time-domain reflectometry with a high precision with respect to the location of hydrogen presence in detection area. In this case, sensing fibers having low bending loss can be used.

Fig. 4 shows an example of a hydrogen presence detecting method according to an embodiment of the invention. Hydrogen detecting method comprises a step S1 of heating, a step S2 of launching light, a step S3 of detecting light, a step S4 of calculating attenuation, a step S5 of quantifying hydrogen presence. This hydrogen detecting method can be performed by hydrogen sensors either of figure 2 or of figure 3.

The step S1 is a step of heating hydrogen sensing fiber 1 so that hydrogen sensing fiber temperature exceeds temperature of outside atmosphere surrounding the hydrogen sensor by at least 10°C, preferably by at least 20°C. In practice, to keep sensing fiber temperature constant, the heating is chosen so as to maintain sensing fiber temperature above outside atmosphere maximal possible temperature by at least 10°C, preferably by at least 20°C.

The sensing time response is all the more shortened by associating a low fiber cladding thickness and a controlled heated temperature of utilization in the range 20°C-300°C, more preferably between 50°C-150°C. The possibility to heat the sensing fiber at a constant temperature at least 20°C higher than the maximal temperature experienced by the sensing fiber in real conditions allows to ensure sensing reliability whatever variations in the outside environment temperature.

Heating step S1 intensity is preferably chosen so that a 1% hydrogen presence in outside atmosphere surrounding the hydrogen sensor can be detected and quantified in less than 1 hour, preferably in less than 10 minutes. Whereas, in prior art detecting hydrogen presence, let alone hydrogen concentration quantifying, could easily take a much longer time, and could for example range from many hours to at least several days.

The step S2 is a step of launching light in a hydrogen sensing fiber included in a hydrogen sensor, at a first wavelength where hydrogen sensing fiber can still undergo reversible hydrogen induced losses. The step S3 is a step of detecting light coming out from hydrogen sensing fiber, at first wavelength. The step S4 is a step of calculating attenuation at first wavelength by comparing detected light power to launched light power at first wavelength. The step S5 is a step of quantifying, directly or indirectly, hydrogen presence in outside atmosphere surrounding the hydrogen sensor from calculated fiber attenuation.

Fig. 5 shows an example of hydrogen sensing fiber pretreatment method according to an embodiment of the invention. Hydrogen sensing fiber pretreatment method successively comprises a step S10 of exposing sensing fiber to a particular atmosphere and a step S20 of out gazing in another atmosphere.

The step S10 is a step of exposing hydrogen sensing fiber, to an atmosphere including a predetermined hydrogen concentration, under a predetermined pressure, under a predetermined temperature, during a first predetermined time. Preferably, predetermined pressure ranges preferably from 1 to 10 atmospheres, more preferably from 2 to 10 atmospheres. Preferably, predetermined temperature ranges preferably from 20°C to 150°C, more preferably from 50°C to 90°C.

Preferably, first predetermined time ranges preferably from 30 hours to 1000 hours. Fist predetermined time which is also the treatment duration of exposing step is defined by the time after which the pre-existing fiber core or fiber cladding defects have fully reacted with hydrogen. This duration will be shorter for higher temperatures and pressure conditions. In case of hydrogen pre-treatment for example, the time needed to reach defect saturation can be defined for example by the saturation of SiOH peak at 1385nm.

The step S20 is a step of out gazing at predetermined conditions. Notably, the temperature is lower or equal to the temperature of fiber pretreatment, preferably at ambient condition during a second predetermined time. Preferably, second predetermined time is preferably at least 2 weeks. This out gazing step S20 can be advantageously combined with sensing fiber storage period.

As for hydrogen concentration is concerned during pretreatment phase, either strict security conditions can be observed or they cannot. In non strictly secured environment, it is better to use a predetermined hydrogen concentration which ranges from 1% to 4%, in order to stay under the concentration threshold of 4% or above leading to an increased risk of explosion. In strictly secured environment, it is better to use a predetermined hydrogen concentration which ranges from 90% to 100%, in order to have the more efficient and the shortest pretreatment duration possible.

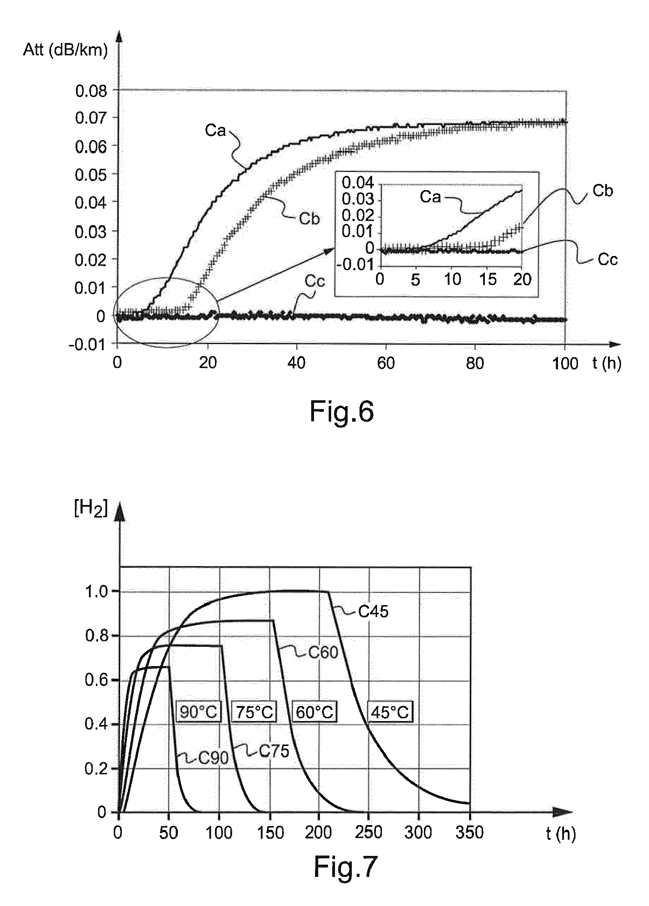

Fig. 6 shows an example of curves showing fiber attenuation increase Att, expressed in dB/km, as a function of time t, expressed in hours h, due to hydrogen diffusion in fiber core in different conditions. Curve Ca represents attenuation peak variation for a sensing fiber according to an embodiment of the invention. Curve Cb represents attenuation peak variation for a sensing fiber according to prior art, that is to say without hydrogen pretreatment. Curve Cc represents attenuation peak non variation for a reference fiber now called "baseline".

Figure 6 illustrates 1242nm peak variation compared to 1310nm used to define baseline, this peak variation indicating molecular hydrogen reversible presence in fiber core. Figure 6 illustrates the effects of hydrogen pretreatment on delay time detection of hydrogen at 1242 nm for the standard 60µm fiber cladding diameter made of glass. Measurement has been performed under 1% hydrogen concentration, pressure value of 1 atmosphere, temperature value of 70°C, during 144 hours. Hydrogen presence in the sensing fiber surroundings is detected thanks to the measurement of a specific peak growth that is related to molecular hydrogen H₂ , this peak spectrally ranging from about 1240nm to about 1244nm, corrected with a baseline which is determined from a specific wavelength in the optical fiber attenuation spectrum, for example at 1310nm where irreversible OH peak has already been saturated by hydrogen pretreatment of sensing fiber, whose level is not impacted by the reversible hydrogen presence. In conclusion, a sensing fiber according to invention presents a much shorter response time for hydrogen presence detection.

Fig. 7 shows an example of curves showing hydrogen presence detection [H₂] (expressed in a normalized relative way) as a function of time t, expressed in hours h, due to hydrogen diffusion in fiber core for different temperatures, expressed in Celsius degrees, of hydrogen sensing fiber. Curve C90 represents hydrogen detection as a function of time for a sensing fiber temperature of 90°C. Curve C75 represents hydrogen detection as a function of time for a sensing fiber temperature of 75°C. Curve C60 represents hydrogen detection as a function of time for a sensing fiber temperature of 60°C. Curve C45 represents hydrogen detection as a function of time for a sensing fiber temperature of 45°C.

Figure 7 illustrates the effects of temperature on the time needed for hydrogen to reach fiber core in the case of a fiber presenting a standard 60µm fiber cladding diameter. Detection level quickly increases until being stabilized; afterwards, it decreases due to disappearance of hydrogen because of out gazing. In conclusion, we can see that the highest the temperature, the quickest the detection and then the shortest the response time of sensing fiber, and this effect is very marked. But we can also see that the highest the temperature, the smallest the attenuation peak increase, and then the less precise the hydrogen quantification, but this effect is rather limited.

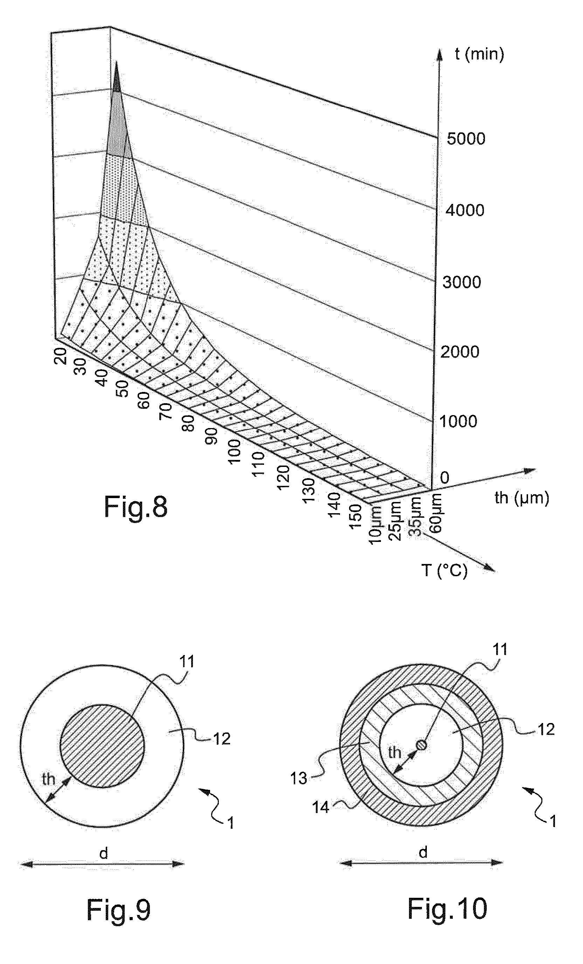

Fig. 8 shows an example of curves showing hydrogen diffusion time t, expressed in minutes min, in fiber core as a function of both hydrogen sensing fiber temperature T, expressed in °C, and hydrogen sensing fiber cladding thickness th, expressed in µm. Figure 8 illustrates the potential delay time reduction when reducing the sensing fiber glass cladding thickness and increasing fiber sensing temperature. One could reach response time at low as several tens of minutes. This response could be even significantly reduced if polymer fiber cladding would be used instead of glass fiber cladding.

Fig. 9 shows another example of a hydrogen sensing fiber according to an embodiment of the invention. Multimode hydrogen sensing fiber 1 presents a fiber core 11 and a fiber cladding 12. Fiber core 11 may be germanium or fluorine doped silica. Fiber cladding 12 may be low index polymer. Multimode sensing fiber 1 could also present one or more thin fiber coatings around fiber cladding 12 to be better protected. But it preferably does not present such coatings in order to accelerate outside hydrogen diffusion towards the core. Sensing fiber 1 presents a diameter d and a fiber cladding thickness th. Sensing fiber 1 diameter d is about 80µm.

Fig. 10 shows still another example of a hydrogen sensing fiber according to an embodiment of the invention. Single mode hydrogen sensing fiber 1 presents a fiber core 11 and a fiber cladding 12. Fiber core 11 may be germanium or fluorine doped silica. Fiber cladding 12 may be pure silica. Single mode sensing fiber 1 may also present one or more fiber coatings around fiber cladding 12 to be better protected, for example a primary coating 13 and a secondary coating 14. Sensing fiber 1 presents a diameter d and a fiber cladding thickness th. Sensing fiber 1 diameter d is about 80µm.

To ensure a fast and reliable response in term of hydrogen content, whatever other environmental changes in the surroundings, in particular temperature changes, a multimode sensing fiber as in figure 9 or a single mode sensing fiber as in figure 10 may present preferred following features, which could be advantageously combined with one another: a concentration of alkali and metallic impurities in the fiber core and fiber cladding below 0.1 ppb, a fiber external diameter between 30 and 150µm, preferably between 30 and 80µm, a glass or polymer fiber cladding, a fiber cladding thickness between 10 and 50 µm, more preferably between 10 and 30 µm. In case of glass fiber cladding, one can use a thin and gas permeable polymer coating resistant to temperature in the range 20°C-300°C, more preferably between 50°C-150°C.

Now, an example of manufacturing steps of a hydrogen sensing fiber according to an embodiment of the invention is described in details. This hydrogen sensing fiber presents 80 µm plastic fiber cladding diameter. The following manufacturing steps are successively realized.

First the core rod is manufactured. A step index profile without clad layers is realized by PCVD (Plasma Chemical Vapor Deposition) technology by successive deposition of germanium and/or fluorine (the co-doping Ge and F allows the core index to be equal to the one of pure silica) doped layers in the inner surface of substrate tube. Second, core rod is collapsed. Third, removal of substrate tube is performed by successive steps of machining and HF etching. Fourth, drawing of core rod down to 50µm fiber core in diameter with in line coating with a low index polymer with a thickness of 15µm is done. The polymer index enables to reach an adequate numerical aperture. Plastic clad with a cladding index in the range of 1.36-1.46 (values given at 850nm) are possible from commercially available resins. Fifth, the fiber is treated in an autoclave under a pressure of 10 atmospheres of pure molecular hydrogen, during 72 hours, at a temperature of 75°C to saturate all glass defects. Sixth, the hydrogen pressure in the autoclave is then replaced by azoth (N₂) under atmospheric pressure during one week at a temperature of 20°C.

Afterwards, the treated sensing fiber can be stocked under air at room temperature during at least two more weeks to reach total molecular hydrogen out gazing. The fiber can then be incorporated in a cable structure of a sensor system allowing continuous heating at a temperature at least 20°C above the maximal environment temperature. In this cable structure, there are means to let hydrogen gas access to direct contact with sensing fiber.

A sensing fiber according to the example above can allow the following hydrogen sensing characteristics. It can measure in a reliable manner the molecular hydrogen concentration in the environment by measuring the specific and reversible peak near at a wavelength of 1242nm +/-2nm. It presents a shortest time response delay thanks to reduced diffusion time of hydrogen towards fiber core. This time delay can even go below one minute at a sensing fiber temperature of 50°C and below 30 seconds at sensing fiber temperature of 65°C. Relation between hydrogen content and reversible hydrogen induced losses (HIL) at a wavelength near 1242nm will remain predictable over time.

The invention has been described with reference to preferred embodiments. However, many variations are possible within the scope of the invention.

## Claims

1. Hydrogen sensing fiber (1), comprising a core (11) and a cladding (12), wherein the fiber is pretreated with a gas able to react with glass defects present in the untreated fiber so that the fiber does not exhibit irreversible hydrogen induced losses at any wavelength after exposure of at least a part of the fiber in an atmosphere comprising Hydrogen.

2. Hydrogen sensing fiber according to claim 1, wherein said gas is Hydrogen.

3. Hydrogen sensing fiber according to any of claims 1 to 2, wherein fiber core (11) is pure silica or silica doped with fluorine.

4. Hydrogen sensing fiber according to any of claims 1 to 3, wherein fiber cladding (12) thickness (th) ranges from 5µm to 30µm.

5. Hydrogen sensing fiber according to any of claims 1 to 4, wherein fiber cladding (12) is made of polymer material.

6. Hydrogen sensing fiber according to any of claims 1 to 5, wherein said hydrogen sensing fiber (1) is a multimode fiber.

7. Hydrogen sensor comprising a hydrogen sensing fiber (1) according to any of preceding claims, said hydrogen sensing fiber (1) being at least partly in contact with an atmosphere coming from outside the hydrogen sensor (9).

8. Hydrogen sensor according to claim 7, further comprising:
- a light source (4) adapted to launch light in said hydrogen sensing fiber (1), at a first wavelength within a spectral range of a reversible attenuation increase due to hydrogen presence in the fiber core (11),
- a light detector (5) adapted to detect light coming out from said hydrogen sensing fiber (1), at said first wavelength.

9. Hydrogen sensor according to claim 8, wherein said light source (4) and said light detector (5) are encompassed in an optical time-domain reflectometer (3).

10. Hydrogen sensor according to claim 8 or 9, wherein said first wavelength is about 1242nm.

11. Hydrogen sensor according to any of claims 7 to 10, further comprising a heater (2) adapted to heat said hydrogen sensing fiber (1).

12. Hydrogen sensor according to any of claims 7 to 11, further comprising a calculator (6) adapted to calculate fiber attenuation by comparing detected light power to launched light power at said first wavelength and to quantify, directly or indirectly, hydrogen presence in an atmosphere surrounding the hydrogen sensor (9) from the calculated fiber attenuation.

13. Hydrogen sensor according to claim 12, wherein said calculator (6) is further adapted to calculate the difference between fiber attenuation at said first wavelength and fiber attenuation at a second wavelength, said hydrogen sensing fiber (1) presenting at said second wavelength an attenuation independent of quantity of hydrogen present in the fiber core (11), and adapted to quantify hydrogen presence in an atmosphere surrounding the hydrogen sensor (9) from said calculated difference.

14. Hydrogen sensor according to claim 12 or 13, further comprising a temperature sensor (7) coupled to said hydrogen sensing fiber (1) and providing said calculator (6) with a signal representing the temperature of said hydrogen sensing fiber (1), and wherein said calculator (6) is adapted to correct calculated fiber attenuation(s) according to hydrogen sensing fiber (1) temperature variations.

15. Hydrogen sensor according to any of claims 7 to 14, wherein the length of the hydrogen sensing fiber (1) is at least 100m, preferably at least 500m, more preferably at least 1000m.

16. Hydrogen sensor according to any of claims 7 to 15, wherein, for at least one point of the hydrogen sensing fiber (1), the lowest from two distances between said point and respectively source (4) and detector (5), is at least 5m.

17. Hydrogen detecting method, comprising:
- a step (S2) of launching light in a hydrogen sensing fiber according to any of claims 1 to 8 included in a hydrogen sensor, at a first wavelength where said hydrogen sensing fiber can still undergo reversible hydrogen induced losses,
- a step (S3) of detecting light coming out from said hydrogen sensing fiber, at said first wavelength,
- a step (S4) of calculating fiber attenuation at said first wavelength by comparing detected light power to launched light power at said first wavelength,
- a step (S5) of quantifying, directly or indirectly, hydrogen presence in outside atmosphere surrounding the hydrogen sensor from calculated fiber attenuation.

18. Hydrogen detecting method according to claim 17, further comprising a step (S1) of heating the hydrogen sensing fiber so that hydrogen sensing fiber temperature exceeds temperature of outside atmosphere surrounding the hydrogen sensor by at least 10°C, preferably by at least 20°C.

19. Hydrogen detecting method according to claim 18, further comprising a step (S1) of heating the hydrogen sensing fiber so that a 1% hydrogen presence in the atmosphere surrounding the hydrogen sensor can be detected and quantified in less than 1 hour, preferably in less than 10 minutes.

20. Hydrogen detecting method according to any of claims 17 to 19, wherein the atmosphere surrounding the hydrogen sensor is radioactive.

21. Hydrogen sensing fiber pretreatment method, successively comprising:
- a step (S10) of exposing said hydrogen sensing fiber, to an atmosphere including a predetermined Hydrogen concentration, under a predetermined pressure, at a predetermined temperature, during a first predetermined time,
- a step (S20) of out gazing at ambient conditions during a second predetermined time.

22. Hydrogen sensing fiber pretreatment method according to claim 21, wherein:
- the predetermined pressure ranges preferably from 1 to 10 atmospheres, more preferably from 2 to 10 atmospheres,
- the predetermined temperature ranges preferably from 20°C to 150°C, more preferably from 50°C to 90°C,
- the first predetermined time ranges preferably from 30 hours to 1000 hours,
- the second predetermined time is preferably at least 2 weeks.

23. Hydrogen sensing fiber pretreatment method according to claim 22 or 23, wherein predetermined hydrogen concentration either ranges from 1% to 4% or ranges from 90% to 100%.
